# FASCICULE DE BREVET EUROPEEN

(11) **EP 1 768 597 B1**
(45) Date de publication et mention de la délivrance du brevet: **01.10.2008**
(21) Numéro de dépôt: 04816317.4
(22) Date de dépôt: 07.07.2004
(51) Int. Cl.: A61C 1/18

(54) **MOTEUR POUR INSTRUMENTS CHIRURGICAUX, NOTAMMENT DENTAIRES**
"MOTOR FÜR OPERATIONSINSTRUMENTE; WIE Z.B. DENTALINSTRUMENTE"
MOTOR FOR SURGICAL INSTRUMENTS, SUCH AS DENTAL INSTRUMENTS

(43) Date de publication de la demande: 04.04.2007
(73) Titulaire: Dassym SA, 2007 Neuchâtel (CH)
(72) Inventeur: GUERNE, Philippe, Henri, CH-2074 Marin (CH); VÖGELI, David, CH-2605 Sonceboz-Sombeval (CH)
(74) Mandataire: P&TS Patents & Technology Surveys SA
(86) Numéro de dépôt international: PCT/EP2004/051397
(87) Numéro de publication internationale: WO 2006/002692

(56) Documents cités:
- CH-A- 676 081
- DE-A1- 3 215 372
- FR-A- 2 707 865
- US-A1- 2003 165 794

## Description

La présente invention concerne un moteur et un attachement pour moteur pour instruments chirurgicaux. La présente invention concerne en particulier un moteur électrique pour instruments chirurgicaux, notamment dentaires, destiné à être connecté à une source d'alimentation au moyen d'un tuyau flexible.

Les moteurs pour instruments chirurgicaux, notamment dentaires, sont de petits moteurs sur lesquels peuvent être placés de manière amovible divers instruments, par exemple des instruments dentaires tels que des contre-angles ou des pièces à main. Le boîtier de ces moteurs est généralement cylindrique et comprend sur une de ses extrémités un support appelé accouplement sur lequel peut être enfiché l'instrument chirurgical à motoriser. Les dimensions de l'accouplement répondent de préférence à des standards, de manière à assurer la compatibilité entre le moteur et un maximum d'instruments différents. A son extrémité opposée, le moteur est généralement connecté à une source d'alimentation au moyen d'un tuyau flexible, permettant ainsi d'alimenter le moteur notamment en électricité tout en offrant à l'instrument une maniabilité optimale.

Le moteur est généralement fixé de manière amovible, par exemple vissé, au tuyau qui est terminé par une pièce appelée attachement. L'attachement comprend une prise par exemple femelle comprenant plusieurs contacts électriques et conduits pour le transport de fluides. Cette prise est destinée à collaborer avec des contacts électriques par exemple mâles et des conduits correspondants sur le moteur.

Dans le cas de moteurs électriques, les contacts électriques sont essentiellement destinés à amener l'énergie électrique nécessaire à l'alimentation du moteur et à la régulation de sa vitesse de rotation, ainsi qu'à l'alimentation d'un moyen d'éclairage, par exemple d'une diode lumineuse, destinée à éclairer le lieu d'intervention de l'instrument. Les conduits pour le transport de fluides sont souvent au nombre de trois ou quatre. Deux conduits sont destinés au transport de l'air et de l'eau qui sont amenés à travers le moteur jusqu'à l'instrument pour y alimenter par exemple un spray destiné à refroidir la zone d'intervention de l'instrument. Généralement, le troisième conduit est destiné à apporter l'air de refroidissement nécessaire au moteur, tandis qu'un éventuel quatrième conduit sert au retour de cet air de refroidissement.

Dans le cas de moteurs à air, l'air sous pression transporté par le troisième conduit est utilisé pour alimenter le moteur. Les contacts électriques de l'attachement peuvent alors ne pas être connectés au moteur. Dans certains cas, seuls deux des contacts électriques disponibles sont connectés pour l'alimentation des moyens d'éclairage.

Dans les moteurs de l'art antérieur, les troisième et quatrième conduits pour le transport des fluides sont parfois disposés sur un même axe passant par l'axe longitudinal du moteur et perpendiculaire à ce dernier. Les contacts électriques, de plus petit diamètre, sont répartis sur d'un côté de ces conduits, tandis que les conduits pour le transport de l'air et de l'eau pour le spray sont placés de l'autre côté. Dans la plupart des moteurs et attachements de l'art antérieur, les contacts électriques sont par exemple disposés sur une première ligne parallèle au diamètre sur lequel sont disposés les troisième et quatrième conduits, les conduits pour l'air et l'eau du spray étant disposés sur une deuxième ligne parallèle à la première. Des exemples de telles dispositions sont par exemple celles définies par les normes ISO 9168.

Le document FR2707865 décrit un connecteur d'irrigation pour instrument chirurgical comprenant des canalisations pour le transport de fluides et la connexion électrique. Le canal pour le transport de fluide et les connexions électriques sont situés de part et d'autre du centre de l'interface de l'attachement proposé.

Le document US2003/0165794 décrit un adaptateur pour appareil médical comprenant également des canalisations pour le transport de fluides et des connexions électriques, dont les répartitions respectives suivant les nombreuses différentes variantes décrites ne sont pas symétriques sur l'interface de l'attachement proposé.

Le document CH676081 décrit une prise d'alimentation d'après les preambules des revendications 1 est 7 pour pièce à main dentaire, comprenant des conduits pour le transport de fluide de gros diamètre et des paires de contacts électriques de plus petits diamètres situés sensiblement sur une même ligne. L'espacement entre les contacts électriques est assez restreint, et la configuration aléatoire sur la face de l'interface rend la vérification de l'état de tous les branchements difficile.

Le document DE3215372 décrit un connecteur hydraulique/pneumatique dans laquelle des canalisations combinent la fonction de conduction de fluides et de conduction électrique. Le connecteur ne concerne pas un moteur et ne contient pas de canalisation centrale de plus gros diamètre pour le transport de fluides, adapté pour son refroidissement par exemple.

Un désavantage de la disposition des éléments de connexion communément admise dans l'art antérieur est que l'espace entre les contacts électriques est faible. Il existe donc un risque que des contacts parasites capacitifs et/ou résistifs s'établissent entre deux contacts électriques voisins. De plus, certains contacts électriques sont plus proches que d'autres du boîtier du moteur, ce qui représente également un risque que des décharges électriques se fassent à travers ce boîtier, menaçant ainsi la sécurité même du patient.

Un autre désavantage des moteurs pour instruments chirurgicaux, notamment dentaires, de l'art antérieur est qu'il est facile d'endommager les contacts électriques, les conduits pour le transport de fluide et/ou le filet extérieur du moyen de fixation de l'attachement lors de la fixation de ce dernier.

Encore un désavantage des moteurs de l'art antérieur est que la vérification visuelle de l'état des éléments de connexion du moteur et/ou de l'attachement correspondant est difficile en raison de l'espacement irrégulier entre les différents éléments de connexion. En particulier, des éléments de connexion mâles pliés ou déformés passent souvent inaperçus.

En outre la norme IEC 60601-1 3^{ème} édition qui entrera prochainement en vigueur prescrira, pour les éléments de connexion des équipements à usage médical, que la distance entre chaque pôle électrique soit au minimum de 2 mm dans l'air et de 4mm de ligne de fuite, ceci afin d'éviter tout couplage galvanique entre les contacts électriques et le boîtier, par exemple. Or, en raison de la disposition des éléments de connexion des moteurs de l'art antérieur, cette norme ne pourra être satisfaite à moins d'augmenter sensiblement le diamètre des moteurs et/ou de diminuer la dimension des contacts, ce qui les rendrait encore plus fragiles.

Un but de la présente invention est par conséquent de proposer un moteur pour instruments chirurgicaux, notamment dentaires, et un attachement ne présentant pas certains désavantages des moteurs et des attachements de l'art antérieur.

Un autre but de l'invention est de proposer un moteur et un attachement offrant une meilleure protection contre les endommagements lors de leur fixation.

Encore un but de l'invention est de proposer un moteur pour instruments chirurgicaux, notamment dentaires, offrant un niveau de sécurité supérieur au niveau de sécurité offert par les moteurs de l'art antérieur.

Un autre but de l'invention est de proposer un moteur pour instruments chirurgicaux, notamment dentaires, répondant aux futures normes de sécurité en la matière qui entreront en vigueur prochainement.

Ces buts sont atteints par un moteur pour instruments chirurgicaux, un attachement et un tuyau possédant les caractéristiques des revendications correspondantes.

Ces buts sont atteints en particulier par un moteur pour instruments chirurgicaux, notamment dentaires, comprenant une interface pour la connexion du moteur à une source d'alimentation, l'interface comprenant des conduits pour le transport de fluides (22-24) et des contacts électriques, les contacts électriques étant disposés sensiblement sur un même arc de cercle autour du centre de l'interface, tous les contacts électriques étant régulièrement espacés entre eux.

Ces buts sont également atteints en particulier par un attachement pour la connexion d'un moteur pour instruments chirurgicaux, notamment dentaires, à une source d'alimentation, comprenant une prise, la prise comprenant des conduits pour le transport de fluides et des contacts électriques, les contacts électriques étant disposés sensiblement sur un même arc de cercle autour du centre de la prise, tous les contacts électriques étant régulièrement espacés entre eux, et par un tuyau pour la connexion d'un moteur pour instruments chirurgicaux, notamment dentaires, à une source d'alimentation, comprenant un tel attachement.

La disposition régulière de tous les contacts électriques autour du centre de l'interface du moteur de l'invention, respectivement autour du centre de la prise de l'attachement, permet d'une part de garantir à chaque contact électrique la même distance jusqu'au boîtier généralement métallique du moteur, et permet d'autre part de réduire les coûts de production et de faciliter l'utilisation du moteur de l'invention grâce à la symétrie de la disposition des éléments de connexion de l'interface.

La présente invention sera mieux comprise à la lecture de la description d'un mode de réalisation préférentiel illustrée par les figures 1 à 3, où:
la figure 1 est une vue en perspective d'un moteur pour instruments chirurgicaux, notamment dentaires, selon une version préférentielle de l'invention, destiné à recevoir d'une part un instrument chirurgical et à être d'autre part attaché à un tuyau relié à une source d'alimentation,
la figure 2 est une vue schématique de face de l'interface du moteur de la figure 1,
la figure 3 est une coupe de l'interface du moteur de la figure 1.

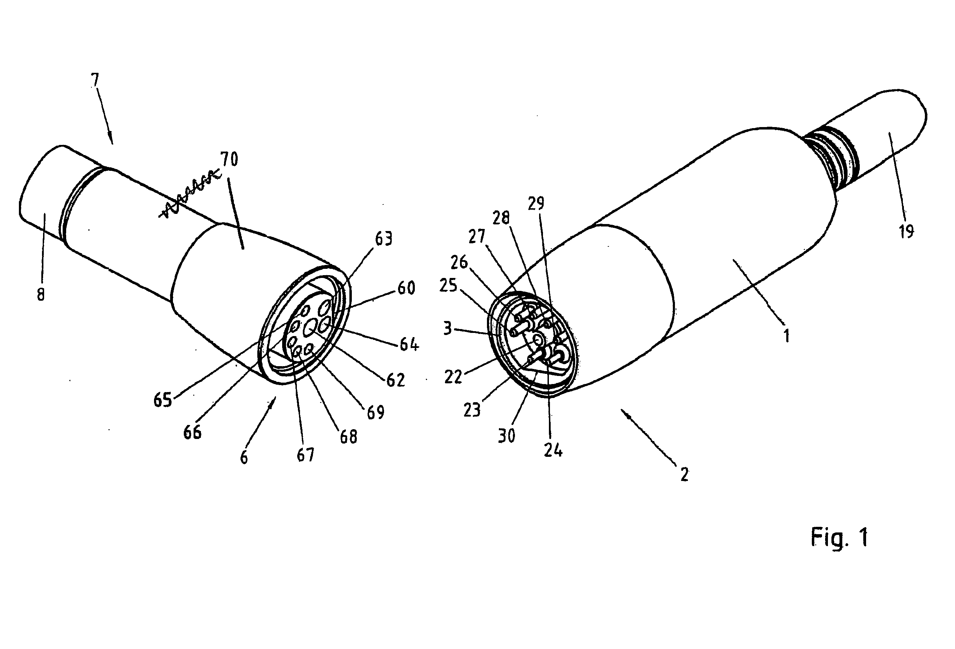

Selon une version préférentielle et en référence à la figure 1, le moteur de l'invention est un moteur électrique pour instruments dentaires alimenté au travers d'un tuyau flexible 8 le reliant à une source d'alimentation non représentée. Le tuyau 8 est terminé par un attachement 7 destiné à être fixé de préférence de manière amovible sur une extrémité 2 du moteur. L'attachement 7 comprend une prise 6 incluant des éléments de connexion par exemple femelles 62-69 destinés à collaborer avec des éléments de connexion mâles 22-29 de l'interface 2 du moteur. Les éléments de connexion 22-29, 62-69 comprennent des contacts électriques 25-29, 65-69 et des conduits pour le transport de fluides 22-24, 62-64.

La prise 6 de l'attachement 7 est destinée à être insérée sur l'interface 2 du moteur afin d'établir les connexions correspondantes entre le moteur et la source d'alimentation non représentée à laquelle le tuyau 8 est relié. Chaque élément de connexion mâle 22-29 de l'interface 2 du moteur est alors de préférence inséré dans l'élément de connexion femelle 62-69 correspondant de l'attachement 7. L'attachement 7 comprend de préférence des moyens de fixation pour assurer le maintien de la connexion entre le tuyau 8 et le moteur. Ces éléments de fixation comprennent par exemple un manchon cylindrique 70 entourant la prise 6 et destiné à être vissé sur l'extrémité 2 du moteur. Le manchon 70 comprend par exemple, sur une partie au moins de sa surface intérieure, un filetage destiné à collaborer avec un pas de vis correspondant sur le moteur.

Sur son extrémité opposée à l'interface 2 destinée à être attachée au tuyau, le moteur de l'invention comprend un accouplement 19 sur lequel peut être fixé un instrument dentaire non représenté. Lorsqu'il est placé sur l'accouplement 19, l'instrument dentaire est entraîné par la rotation du moteur au moyen d'un arbre de transmission non représenté collaborant avec des moyens de transmission dans le manche de l'instrument. L'instrument dentaire est de préférence fixé de manière amovible sur le moteur de manière à pouvoir être changé facilement par un autre instrument en cas de besoin.

Selon une version préférentielle de l'invention, les connexions entre le moteur et l'attachement 7 comprennent trois conduits pour le transport de fluides 22-24, 62-64 et cinq contacts électriques 25-29, 65-69.

Selon une version préférentielle, le moteur de l'invention est un moteur électrique. Une partie au moins des contacts électriques 25-29, 65-69 servent alors à amener l'énergie électrique pour l'alimentation du moteur et les signaux analogiques ou numériques nécessaires à la régulation de sa vitesse de rotation et de son couple. Le moteur est par exemple un moteur triphasé et trois contacts électriques 25, 27, 29 resp. 65, 67, 69 servent à son alimentation. Les signaux de régulation sont de préférence modulés sur les tensions d'alimentation et utilisent ainsi les mêmes contacts électriques 25, 27, 29 resp. 65, 67, 69 que ces dernières.

Selon une autre variante de l'invention, le moteur est un moteur à air alimenté par de l'air comprimé transporté par exemple dans le conduit central 22, 62.

Une paire de contacts électriques est de préférence réservée pour l'alimentation d'une source lumineuse non représentée, par exemple d'une diode lumineuse, placée de préférence à la base de l'accouplement 19 et dont la lumière est par exemple conduite à travers l'instrument jusqu'à son extrémité afin d'éclairer l'endroit, par exemple la dent, qui sera traité par l'instrument.

Selon une variante de l'invention, des signaux de données destinés par exemple au système de régulation du moteur et/ou pour l'activation d'éléments ou d'options supplémentaires sont modulés sur la tension d'alimentation de la source lumineuse et/ou du moteur et utilisent ainsi les mêmes contacts électriques que ces dernières.

Un premier conduit 23, 63 pour le transport de fluide transporte de préférence de l'air comprimé, tandis qu'un deuxième conduit 24, 64 transporte de l'eau. L'air et l'eau transportés par ces deux conduits 23, 63 et 24, 64 sont destinés à être amenés jusqu'à l'instrument dentaire afin de former par exemple à son extrémité un spray pour refroidir la zone de travail de l'instrument. Le spray est ainsi formé d'un mélange d'air et d'eau, les proportions entre ces deux éléments pouvant varier sensiblement selon les besoins, l'instruments utilisé, etc. Toutes les proportions sont en effet envisageables, allant d'un jet d'eau uniquement à un jet d'air seul dans le cas par exemple où la zone d'intervention de l'instrument ne doit pas être mouillée.

L'interface 2 du moteur et la prise 6 de l'attachement 7 comprennent de préférence un conduit central 22, resp. 62 pour le transport d'un fluide destiné au refroidissement du moteur électrique et, en cas de nécessité, de l'instrument entraîné par ce dernier. Le fluide de refroidissement est de préférence de l'air qui est pulsé dans le boîtier 1, circule entre les différents éléments électromécaniques du moteur et éventuellement de l'instrument, puis ressort par des ouvertures non représentées ménagées dans le boîtier 1 et/ou dans l'instrument.

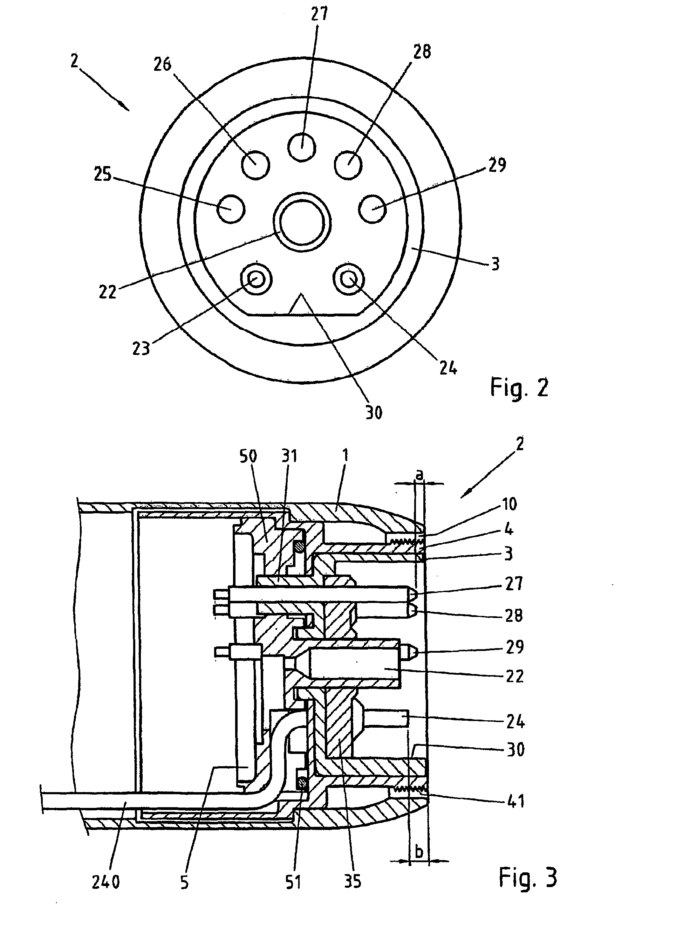

La répartition des éléments de connexion de l'interface 2 est illustrée à la figure 2 qui est une vue schématique de face de l'extrémité correspondante du moteur.

Selon l'invention, tous les éléments de connexion 23-29 sauf le conduit central 22 ont sensiblement le même diamètre extérieur et la même forme. Le diamètre extérieur des conduits 23, 24 pour le transport de l'air et de l'eau destinés par exemple à la formation du spray étant sensiblement identique au diamètre extérieur des contacts électriques 25-29, tous ces éléments de connexion 23-29 peuvent être répartis de manière optimale, de préférence régulièrement, autour du centre de l'interface 2. Seul le diamètre du conduit central 22 est de préférence sensiblement plus grand que celui des autres éléments de connexion 23-29 afin de permettre une amenée d'air suffisante pour refroidir le moteur quelle que soit son utilisation.

Les éléments de connexion 23-29 sont de préférence répartis régulièrement autour du centre de l'interface 2. Les contacts électriques 25-29 occupent par exemple une moitié de l'interface tandis que les conduits 23, 24 pour l'air et l'eau destinés par exemple à la formation du spray occupent l'autre moitié. Les contacts électriques 25-29 sont ainsi par exemple disposés sensiblement sur même un arc de cercle dont le centre de rotation se situe sensiblement au centre de l'interface 2, l'espacement entre deux contacts électriques voisins étant de préférence identique pour chaque paire de contacts. Les conduits 23, 24 pour l'air et l'eau destinés par exemple à la formation du spray sont de préférence également disposés sensiblement sur un arc de cercle centré sur le centre de l'interface 2, de préférence sur le même arc de cercle que celui sur lequel sont disposés les contacts électriques 25-29. Ainsi qu'expliqué précédemment, le centre de l'interface 2 est occupé par le conduit central 22 pour le transport du fluide de refroidissement et dont le diamètre extérieur est de préférence sensiblement supérieur au diamètre extérieur des autres éléments de connexion.

Pour des raisons évidentes de conduction électrique, les contacts électriques 25-29 sont en un matériau électriquement conducteur, de préférence métallique. Ils forment ainsi un premier pôle électrique au sens de la norme IEC 60601-1 3^{ème} édition, par exemple. Le boîtier 1 du moteur, de préférence également métallique et électriquement conducteur forme un deuxième pôle électrique ne possédant aucun couplage galvanique avec le premier. Selon une variante préférentielle, les conduits 23, 24 pour l'air et l'eau sont de préférence également métalliques afin de leur assurer une meilleure tenue mécanique et une plus grande longévité. Ils forment ainsi un pôle électrique qui est, dans une version préférentielle de l'invention et pour les raisons évoquées plus bas, électriquement connecté au boîtier 1. Selon cette variante préférentielle de l'invention, les cinq contacts électriques 25-29 forment un premier pôle électrique tandis que les deux conduits 23, 24 pour l'air et l'eau forment un deuxième pôle électrique avec le boîtier 1 du moteur.

Grâce à la répartition des éléments de connexion 23-29 selon l'invention, la distance entre chaque contact électrique 25-29 et le boîtier métallique 1 du moteur est identique. La longueur de la ligne de fuite jusqu'au boîtier 1 est donc identique pour chaque contact électrique 25-29, offrant ainsi une sécurité optimale et identique à chaque contact électrique 25-29 contre tout couplage électrique avec le boîtier 1 du moteur. D'autre part, la répartition des contacts électriques 25-29 sur un même arc de cercle permet notamment de réduire le diamètre extérieur du moteur sans devoir diminuer sensiblement la distance séparant les contacts électriques entre eux. Pratiquement, le diamètre de l'interface 2 est déterminé par la distance minimale devant être conservée entre les contacts électriques 25-29 et le boîtier 1 et/ou par la distance minimale nécessaire entre les contacts électriques 25-29 et le conduit central 22 si ce dernier est électriquement conducteur.

Selon une variante préférentielle de l'invention, le conduit central 22 pour le transport du fluide de refroidissement est en un matériau synthétique et non conducteur, de sorte qu'il n'existe pas de risque de couplage galvanique entre ce dernier et les contacts électriques 25-29. Il ne constitue pas de pôle électrique au sens par exemple de la norme IEC 60601-1 3^{ème} édition.

Afin d'augmenter encore la longueur de la ligne de fuite entre les contacts électriques 25-29 et le boîtier 1 et/ou les conduits 23, 24 pour le transport de l'air et de l'eau, la surface intérieure de l'interface 2 est de préférence recouverte d'une matière synthétique non conductrice 3. Cette matière synthétique est par exemple une pièce d'isolation 3 en matière plastique disposée dans le fond de l'interface 2 et comprenant une paroi essentiellement circulaire formant la paroi intérieure de l'interface 2.

Afin d'éviter une mauvaise connexion de l'attachement sur l'interface 2 du moteur, cette dernière comprend de préférence des éléments de détrompage empêchant toute fixation de l'attachement si sa position angulaire par rapport à l'interface 2 n'est par correcte. Les éléments de détrompage comprennent par exemple un détrompeur 30 formé par une surface plate sur la paroi intérieure essentiellement circulaire de l'interface 2, destinée à collaborer avec une surface plate 60 sur la paroi extérieure de la prise 6 de l'attachement 7 (figure 1).

D'autres éléments de détrompage sont également envisageables dans le cadre de l'invention. Il est en particulier possible d'imaginer une ou plusieurs rainures de largeurs différentes et/ou de disposition irrégulière formées sur la paroi intérieure de l'interface et/ou sur la paroi extérieure de la prise, destinées à collaborer avec un ou plusieurs ergots correspondants formés respectivement sur la paroi extérieure de la prise et/ou sur la paroi intérieure de l'interface.

L'homme du métier comprendra que la disposition des éléments de connexion femelles 62-69 de la prise 6 de l'attachement 7 de l'invention (figure 1) correspond à la disposition expliquée plus haut pour les éléments de connexion 22-29 mâles de l'interface 2 du moteur, de sorte que les connexions soient correctement établies entre les différents éléments lorsque l'attachement 7 est fixé au moteur. La répartition des éléments de connexion femelles 62-69 selon le principe de l'invention procure en outre essentiellement les mêmes effets avantageux que ceux produits par la répartition selon l'invention des éléments de connexions mâles 22-29.

La figure 3 est une coupe de l'interface 2 du moteur de l'invention à travers son centre. Ainsi qu'évoqué précédemment, le moteur est protégé sur sa périphérie par un boîtier 1, de préférence métallique et électriquement isolé. Le boîtier 1 confère au moteur la forme et l'aspect extérieurs désirés. Le boîtier 1 est de préférence rigide. Il peut cependant comprendre des éléments non représentés en matière plus ou moins souple afin par exemple d'offrir un meilleur confort d'utilisation.

Un élément de fixation 4 essentiellement cylindrique est de référence inséré dans le boîtier 1 et maintenu dans une position déterminée contre la paroi intérieure de ce dernier. A l'extrémité du boîtier 1, un espace 10 est de préférence ménagé entre la paroi extérieure de l'élément de fixation 4 et la paroi intérieure du boîtier 1. Lorsque le tuyau est fixé au moteur, une partie au moins du manchon 70 de l'attachement 7 (figure 1) est inséré dans cet espace 10. Un pas de vis 41 est de préférence formé sur la paroi extérieure de l'élément de fixation 4, destiné à collaborer avec le filetage formé sur la paroi intérieure du manchon 70. Le manchon 70 et l'élément de fixation 4 sont de préférence métalliques de manière à assurer un bon maintien mécanique de la connexion et à assurer que le boîtier 1 et le manchon 70 soient correctement connectés électriquement entre eux, l'élément de fixation 4 étant de préférence en contact électrique avec le boîtier 1. L'élément de fixation 4 fait ainsi partie du même pôle électrique que le boîtier 1.

Une pièce d'isolation 3 en matière non conductrice électriquement est de préférence insérée dans le fond et contre les parois intérieures de l'interface 2 afin d'augmenter la longueur des lignes de fuite entre les contacts électriques 25-29 et l'élément de fixation métallique 4, améliorant ainsi l'isolation entre les contacts électriques et le boîtier 1 du moteur. La pièce d'isolation 3 est par exemple une pièce en matière synthétique, par exemple moulée ou injectée. Elle comprend de préférence des ouvertures sur son fond permettant le passage des contacts électriques 25-29 et des conduits 23, 24 pour le transport de l'air et de l'eau vers l'intérieur du boîtier. Les ouvertures pour le passage des contacts électriques sont de préférence chacune prolongées dans le boîtier du moteur par un cylindre 31 formé dans la même pièce d'isolation 3 et offrant aux contacts électriques une bonne isolation électrique par rapport aux autres éléments se trouvant à l'intérieur du boîtier 1.

Les contacts électriques 25-29 sont par exemple formés de tiges électriquement conductrices et fixées sur un circuit imprimé 5 à l'intérieur du boîtier 1. Les différents éléments du moteur nécessitant une alimentation en électricité tels que par exemple le moteur lui-même, la source lumineuse, etc., sont à leur tour également connectés au circuit imprimé 5, par exemple à l'aide de conducteurs électriques souples isolés. Le circuit imprimé 5 fait ainsi office de point de distribution à l'intérieur du moteur. Le circuit imprimé 5 est de préférence maintenu sur un support 50 non conducteur, par exemple en matière synthétique moulée ou injectée, lui-même inséré dans l'élément de fixation 4. De préférence, le conduit central 22 de l'interface 2 permettant l'arrivée du fluide de refroidissement dans le boîtier 1 du moteur est formé par une ouverture cylindrique du support 50 traversant l'élément de fixation 4 et la pièce d'isolation 3.

Selon une variante préférentielle de l'invention, le support 50 est maintenu contre la paroi intérieure de l'élément de fixation 4 par l'appui des autres éléments non représentés insérés par la suite dans le boîtier 1. L'ensemble est maintenu en légère compression par la fermeture du boîtier. Un joint torique 51 est de préférence inséré entre le support 50 et l'élément de fixation 4 afin d'assurer une force de compression minimale malgré d'éventuelles imprécisions dans les dimensions des différentes pièces.

De préférence, le support 50 est en matière synthétique non conductrice électriquement. Il ne représente donc pas un pôle électrique au sens de la norme IEC 60601-1 3^{ème} édition, par exemple. Le conduit 22 pour le transport du fluide de refroidissement étant de préférence formé directement dans le support 50, il est de préférence également en matière synthétique non conductrice, ainsi qu'évoqué précédemment.

Les conduits 23, 24 pour le transport de l'air et de l'eau sont de préférence reliés chacun à un tuyau 240 permettant le transport du fluide correspondant à travers le moteur jusqu'à l'accouplement où il sera, le cas échéant, introduit et guidé dans l'instrument fixé sur le moteur. Les tuyaux sont de préférence des tuyaux métalliques rigides offrant une meilleure tenue mécanique que par exemple des tuyaux souples en caoutchouc. Les tuyaux mécaniques offrent en particulier une meilleure résistance aux stérilisations auxquelles doit régulièrement être soumis le moteur.

Le fond de l'interface 2 est de préférence recouvert d'une d'un joint plat 35 en matière synthétique souple assurant une bonne étanchéité entre les connexions lorsque la prise est fixée sur l'interface 2. Le joint plat 35 est alors de préférence légèrement comprimé entre la prise et la pièce d'isolation 3.

La longueur des éléments de connexion 22-29 depuis le fond de l'interface 2 en direction de l'extrémité du moteur est de préférence inférieure à la longueur de la paroi intérieure de l'interface 2, de sorte que, lors de la connexion du tuyau sur le moteur, la prise de l'attachement entre d'abord en contact avec les éléments mécaniques de l'interface 2, de préférence au moins avec les éléments de détrompage 30, avant d'entrer en contact avec les éléments de connexion 22-29 qui sont plus fragiles et pourraient être endommagés par une insertion incorrecte de la prise. Les éléments de connexion sont ainsi protégés contre toute contrainte mécanique indésirable lors de la connexion du tuyau au moteur par les bords extérieurs de l'interface 2 dont la longueur dépasse d'une distance a la longueur des contacts électriques 25-29 et d'une distance b la longueur des conduits pour le transport des fluides 22-24. Les distances a et b sont de préférence différentes afin de faciliter la connexion du moteur au tuyau, l'engagement successif des différents éléments de connexion dans la prise étant plus facile que leur engagement simultané. Dans l'exemple illustré, la distance b est par exemple supérieure à la distance a. Lors du branchement de la prise sur l'interface 2, les contacts électriques 25-29 sont ainsi engagés avant les conduits 22-24 pour le transport de fluides.

Selon une version préférentielle de l'invention, le moteur est entièrement stérilisable. Le boîtier 1 est de préférence fermé par une douille détachable contenant le moteur et formant l'accouplement. Lors de la stérilisation, la douille est par exemple dévissée du reste du boîtier et placée avec le moteur électrique qu'elle contient dans le stérilisateur. Selon une autre variante, le moteur complet, y compris son boîtier fermé, est détaché du tuyau et placé dans le stérilisateur, permettant ainsi également la stérilisation de l'interface 2.

D'autres constructions de l'interface 2 du moteur de l'invention sont également possible. Il est en particulier possible d'imaginer d'autres éléments que le circuit imprimé 5 pour fixer les contacts électriques 25-29 dans l'interface 2 et pour permettre ensuite la redistribution de l'énergie électrique dans le moteur. Il est également possible d'imaginer d'autres formes de réalisation pour l'élément de fixation 4, une partie de ses fonctions, en particulier ses fonctions de fixation du manchon de l'attachement, pouvant en particulier être assurée par le boîtier 1 lui-même.

Dans la description ci-dessus, l'interface 2 et ses éléments de connexion sont généralement cylindriques. Bien que cette forme de réalisation présente l'avantage d'être de réalisation simplifiée, d'autres formes sont également possibles dans le cadre de l'invention. Il est en particulier possible d'imaginer une interface comprenant des éléments de connexion hexagonaux, octogonaux, etc.

Selon la version préférentielle de l'invention, tous les contacts électriques 25-29 sont identiques entre eux, ainsi que les conduits pour le transport de l'air et de l'eau. De plus, la répartition de ces éléments est symétrique par rapport au centre de l'interface. Une telle réalisation permet d'une part de réduire au minimum le nombre de pièces différentes constituant l'interface 2, et d'autre part de simplifier la réalisation de ces pièces grâce à leur forme symétrique. Les coûts de production et la complexité de l'interface 2 sont donc minimisés. La symétrie et la simplicité de l'interface permet en outre de faciliter son contrôle, en particulier son contrôle visuel par exemple avant le branchement du tuyau.

## Revendications

1. Moteur pour instruments chirurgicaux, notamment dentaires, comprenant une interface (2) pour la connexion dudit moteur à une source d'alimentation, ladite interface (2) comprenant des conduits pour le transport de fluides (22-24) et des contacts électriques (25-29), lesdits contacts électriques (25-29) étant disposés sensiblement sur un même arc de cercle autour du centre de ladite interface (2), tous lesdits contacts électriques (25-29) étant régulièrement espacés entre eux, **caractérisé en ce que:**
un desdits conduits pour le transport de fluides (22) est disposé au centre de ladite interface (2),
les autres dits conduits pour le transport de fluides (23, 24) sont disposés sensiblement sur un même arc de cercle autour du centre de ladite interface (2).

2. Moteur selon la revendication précédente, lesdits contacts électriques (25-29) étant au nombre de cinq, ledit conduit (22) disposé au centre de ladite interface servant au transport d'un fluide de refroidissement et les autres dits conduits (23, 24) servant respectivement au transport d'eau et d'air comprimé.

3. Moteur selon la revendication précédente, lesdits contacts électriques (25-29) et lesdits deux conduits (23, 24) pour le transport d'eau et d'air comprimé étant constitués d'éléments de connexion mâles ayant sensiblement le même diamètre extérieur.

4. Moteur selon l'une des revendications 2 ou 3, lesdits deux conduits (23, 24) pour le transport d'eau et d'air comprimé étant disposés sur le même arc de cercle autour du centre de ladite interface (2) que lesdits contacts électriques (25-29).

5. Moteur selon l'une des revendications précédentes, ladite interface (2) comprenant au moins un élément de détrompage pour assurer une connexion mécanique correcte dudit moteur à une source d'alimentation.

6. Moteur selon l'une des revendications précédentes, ladite interface (2) comprenant une pièce d'isolation (3) en matière non conductrice électriquement pour augmenter la longueur des lignes de fuite entre lesdits contacts électriques (25-29) et un boîtier (1) dudit moteur.

7. Attachement (7) pour la connexion d'un moteur pour instruments chirurgicaux, notamment dentaires, à une source d'alimentation, comprenant une prise (6), ladite prise (6) comprenant des conduits pour le transport de fluides (62-64) et des contacts électriques (65-69), lesdits contacts électriques (65-69) étant disposés sensiblement sur un même arc de cercle autour du centre de ladite prise (6), tous lesdits contacts électriques (65-69) étant régulièrement espacés entre eux, **caractérisé en ce que**:
un desdits conduits pour le transport de fluides (62) est disposé au centre de ladite prise (6),
les autres dits conduits pour le transport de fluides (63, 64) sont disposés sensiblement sur un même arc de cercle autour du centre de ladite prise (6).

8. Attachement (7) selon la revendication précédente, comprenant lesdits contacts électriques (65-69) étant au nombre de cinq, ledit conduit (62) disposé au centre de ladite interface servant au transport d'un fluide de refroidissement et les autres dits conduits (63, 64) servant au transport d'eau et d'air comprimé.

9. Attachement (7) selon la revendication précédente, lesdits contacts électriques (65-69) et lesdits deux conduits (63, 64) pour le transport d'eau et d'air comprimé étant constitués d'éléments de connexion femelles ayant sensiblement le même diamètre intérieur.

10. Attachement (7) selon l'une des revendications 8 ou 9, lesdits deux conduits (63, 64) pour le transport d'eau et d'air comprimé étant disposés sur le même arc de cercle autour du centre de ladite prise (6) que lesdits contacts électriques (65-69).

11. Attachement (7) selon l'une des revendications précédentes, ladite prise (6) comprenant au moins un élément de détrompage pour assurer une connexion mécanique correcte dudit attachement (7) à un moteur pour instruments chirurgicaux, notamment dentaires.

12. Tuyau pour la connexion d'un moteur pour instruments chirurgicaux, notamment dentaires, à une source d'alimentation, comprenant un attachement selon l'une des revendications 7 à 11.

## Claims

1. Motor for surgical instruments, notably dental instruments, comprising an interface (2) for connecting said motor to a power source, said interface (2) having conduits for transporting fluids (22-24) and electric contacts (25-29), said electric contacts (25-29) being arranged more or less on a same arc of circle around the centre of said interface (2), all said electric contacts (25-29) being regularly spaced between one another,
**characterized in that:**
one of said conduits for transporting fluids (22) is placed at the centre of said interface (2),
the other said conduits for transporting fluids (23, 24) are placed more or less on a same arc of circle around the centre of said interface (2).

2. Motor according to the preceding claim, said electric contacts (25-29) numbering five, said conduit (22) placed at the centre of said interface serving to transport a cooling fluid and the other said conduits (23, 24) serving to the transport of water and compressed air respectively.

3. Motor according to the preceding claim, said electric contacts (25-29) and said two conduits (23, 24) for transporting water and compressed air being constituted of male connection elements having more or less the same outer diameter.

4. Motor according to one of the claims 2 or 3, said two conduits (23, 24) for transporting water and compressed air being placed on the same arc of circle around the centre of said interface (2) as said electric contacts (25-29).

5. Motor according to one of the preceding claims, said interface (2) comprising at least one keying element for ensuring a correct mechanical connection of said motor to a power source.

6. Motor according to one of the preceding claims, said interface (2) comprising an insulation part (3) of non electrically conducting element to increase the length of the creepage distance between said electric contacts (25-29) and a casing (1) of said motor.

7. Fitting (7) for connecting a motor for surgical instruments, notably dental instruments, to a power source, comprising a socket (6), said socket (6) comprising conduits for transporting fluids (62-64) and electric contacts (65-69), said electric contacts (65-69) being placed more or less on a same arc of circle around the centre of said socket (6), all said electric contacts (65-69) being regularly spaced between one another,
**characterized in that**:
one of said conduits for transporting fluids (62) is placed at the centre of said socket (6),
the other said conduits for transporting fluids (63, 64) are placed more or less on a same arc of circle around the centre of said socket (6).

8. Fitting (7) according to the preceding claim, comprising said electric contacts (65-69) numbering five, said conduit (62) placed at the centre of said interface serving to transport a cooling fluid and the other said conduits (63, 64) serving to the transport of water and compressed air.

9. Fitting (7) according to the preceding claim, said electric contacts (65-69) and said two conduits (63, 64) for transporting water and compressed air being constituted of female connection elements having more or less the same inner diameter.

10. Fitting (7) according to one of the claims 8 or 9, said two conduits (63, 64) for transporting water and compressed air being placed on the same arc of circle around the centre of said socket (6) as said electric contacts (65-69).

11. Fitting (7) according to one of the preceding claims, said socket (6) comprising at least one keying element for ensuring a correct mechanical connection of said fitting (7) to a motor for surgical instruments, notably dental instruments.

12. Tube for connecting a motor for surgical instruments, notably dental instruments, to a power source, comprising a fitting according to one of the claims 7 to 11.

## Patentansprüche

1. Motor für chirurgische Instrumente, insbesondere Dentalinstrumente, mit einer Schnittstelle (2) für die Verbindung des besagten Motors an einer Stromquelle, wobei die besagte Schnittstelle (2) Leitungsröhren für den Transport von Flüssigkeiten (22-24) und elektrische Kontakte (25-29) umfasst, wobei die besagten elektrischen Kontakte (25-29) auf einen mehr oder weniger gleichen Kreisbogen um das Zentrum der besagten Schnittstelle (2) angeordnet sind, wobei die besagten elektrischen Kontakte (25-29) in regelmässigen Abständen zu einander liegen, **dadurch gekennzeichnet, dass**
eine der Leitungsröhren für den Transport von Flüssigkeiten (22) am Zentrum der besagten Schnittstelle (2) angeordnet ist,
wobei die anderen besagten Leitungsröhren für den Transport von Flüssigkeiten (23, 24) auf einem mehr oder weniger gleichen Kreisbogen um das Zentrum der besagten Schnittstelle (2) angeordnet sind.

2. Motor gemäss dem vorhergehenden Anspruch, wobei die besagten elektrischen Kontakte (25-29) fünf sind, wobei die besagte am Zentrum der besagten Schnittstelle angeordnete Leitungsröhre (22) für den Transport einer Kühlungsflüssigkeit dient, und die anderen besagten Leitungsröhren (23, 24) für den Transport von Wasser bzw. von Druckluft dienen.

3. Motor gemäss dem vorhergehenden Anspruch, wobei die besagten elektrischen Kontakte (25-29) und die besagten beiden Leitungsröhren (23, 24) für den Transport von Wasser und Druckluft aus männlichen Verbindungselementen bestehen, welche mehr oder weniger den gleichen Aussendurchmesser aufweisen.

4. Motor gemäss einem der vorhergehenden Ansprüche 2 oder 3, wobei die besagten beiden Leitungsröhren (23, 24) für den Transport von Wasser und Druckluft auf dem gleichen Kreisbogen um das Zentrum der besagten Schnittstelle (2) wie die besagten elektrischen Kontakte (25-29) angeordnet sind.

5. Motor gemäss einem der vorhergehenden Ansprüche, wobei die besagte Schnittstelle (2) mindestens ein Führungselement umfasst, um eine korrekte mechanische Verbindung des besagten Motors mit einer Stromquelle zu gewährleisten.

6. Motor gemäss einem der vorhergehenden Ansprüche, wobei die besagte Schnittstelle 82) einen Isolationsteil (3) aus nicht elektrisch leitendem Material umfasst, um die Länge des Kriechwegs zwischen den besagten elektrischen Kontakten (25-29) und einem Gehäuse (1) des besagten Motors zu verlängern.

7. Aufsatz (7) für die Verbindung eines Motors für chirurgische Instrumente, insbesondere Dentalinstrumente, mit einer Stromquelle, mit einem Anschluss, wobei der besagte Anschluss (6) Leitungsröhren für den Transport von Flüssigkeiten (62-64) und elektrische Kontakte (65-69) umfasst, wobei die besagten elektrischen Kontakte (65-69) auf einem mehr oder weniger gleichen Kreisbogen um das Zentrum des besagten Anschlusses (6) angeordnet sind, wobei alle besagten elektrischen Kontakte (65-69) in regelmässigen Abständen zu einander liegen, **dadurch gekennzeichnet, dass**
eine der Leitungsröhren für den Transport von Flüssigkeiten (62) am Zentrum des besagten Anschlusses (6) angeordnet ist,
wobei die anderen besagten Leitungsröhren für den Transport von Flüssigkeiten (63, 64) auf einem mehr oder weniger gleichen Kreisbogen um das Zentrum des besagten Anschlusses (6) angeordnet sind.

8. Aufsatz (7) gemäss dem vorhergehenden Anspruch, wobei die besagten elektrischen Kontakte (65-69) fünf sind, wobei die besagte am Zentrum der besagten Schnittstelle angeordnete Leitungsröhre (62) für den Transport einer Kühlungsflüssigkeit dient, und die anderen besagten Leitungsröhren (63, 64) für den Transport von Wasser bzw. von Druckluft dienen.

9. Aufsatz (7) gemäss dem vorhergehenden Anspruch, wobei die besagten elektrischen Kontakte (65-69) und die besagten beiden Leitungsröhren (63, 64) für den Transport von Wasser und Druckluft aus weiblichen Verbindungselementen bestehen, welche mehr oder weniger den gleichen Innendurchmesser aufweisen.

10. Aufsatz (7) gemäss einem der Ansprüche 8 oder 9, wobei die besagten beiden Leitungsröhren (63, 64) für den Transport von Wasser und Druckluft auf dem gleichen Kreisbogen um das Zentrum des besagten Anschlusses (6) wie die besagten elektrischen Kontakte (65-69) angeordnet sind.

11. Aufsatz (7) gemäss einem der vorhergehenden Ansprüche, wobei der besagte Anschluss (6) mindestens ein Führungselement umfasst, um eine korrekte mechanische Verbindung des besagten Aufsatzes (7) mit einem Motor für chirurgische Instrumente, insbesondere Dentalinstrumente, zu gewährleisten.

12. Schlauch für die Verbindung eines Motors für chirurgische Instrumente, insbesondere Dentalinstrumente, mit einer Stromquelle, mit einem Aufsatz gemäss einem der Ansprüche 7 bis 11.
